# EUROPÄISCHE PATENTANMELDUNG

(11) **EP 3 315 491 A1**
(43) Veröffentlichungstag der Anmeldung: **02.05.2018**
(21) Anmeldenummer: 17197122.9
(22) Anmeldetag: 18.10.2017
(51) Int. Cl.: C07D 231/08, C07D 231/12, C07D 231/14, C07D 487/04, C07C 245/14, C07D 213/26, C07C 17/32, C07C 22/08

(54) **VERFAHREN ZUR HERSTELLUNG FLUORIERTER DIAZOALKANE**

(30) Priorität: 28.10.2016 DE 102016120688
(71) Anmelder: Rheinisch-Westfälische Technische Hochschule (RWTH) Aachen, 52056 Aachen (DE)
(72) Erfinder: Königs, Rene, 41812 Erkelenz (DE); Hock, Katharina Julia, 52072 Aachen (DE); Mertens, Lukas, 52249 Eschweiler (DE)
(74) Vertreter: Michalski Hüttermann & Partner Patentanwälte mbB

(57) **Zusammenfassung**

Die Erfindung betrifft ein Verfahren zur Herstellung eines fluorierten Diazoalkans, wobei es sich bei dem Verfahren um ein kontinuierliches Verfahren handelt und ein β,β-Difluoralkylamin mit einem organischen Nitrit in einem Reaktor umgesetzt wird, wobei das β,β-Difluoralkylamin und das organische Nitrit in getrennten Gefäßen vorgelegt werden, sowie die Verwendung des Verfahren zur Herstellung einer fluoralkyl-substituierten Verbindung.

## Beschreibung

Die Erfindung betrifft ein Verfahren zur Herstellung fluorierter Diazoalkane.

Die Synthese funktionalisierter, fluorierter Verbindungen, beispielsweise von Heterocyclen, ist insbesondere für die pharmazeutische und agrochemische Industrie von hoher Bedeutung, da fluorierte Verbindungen häufig begehrte Eigenschaften wie eine hohe Lipophilie und metabolische Stabilität aufweisen. Eine der Schwierigkeiten bei der Synthese fluorierter organischer Verbindungen liegt jedoch in der Verwendung hochreaktiver und entsprechend gefährlicher Fluorierungsreagenzien. Ein weiterer Nachteil bekannter präparativer Routen liegt darin, dass diese häufig mehrschrittig sind, und bei jedem Schritt Verluste, beispielsweise durch notwendige Aufarbeitungs- und Reinigungsschritte, die Ausbeute der gewünschten Verbindung verringern. Daher ist das Auffinden neuer bzw. Verbessern bekannter Synthesestrategien für den Einbau von Fluoratomen in organische Verbindungen in der präparativen Chemie von hoher Bedeutung.

Eine ökonomische Methode für die direkte Synthese fluoralkyl-substituierter Pyrazole bietet die [2+3]-Cycloaddition fluoralkyl-substituierter Diazomethane mit Olefinen oder Alkinen. Eine Synthese der hierzu notwendigen entsprechend fluoralkyl-substituierten Diazomethane in einer säurekatalysierten Reaktion unter Verwendung von Nitrit ist bekannt. Herkömmliche, diskontinuierliche Verfahren benötigen aufgrund der Explosionsgefahr der entstehenden Diazoverbindungen jedoch strikte Sicherheitsvorkehrungen wie die Verwendung einer speziellen Glasausrüstung, wodurch die Synthese größerer Mengen erschwert wird. Ein kontinuierliches Verfahren zur Herstellung fluoralkyl-substituierter Diazomethane und deren nachfolgende Verwendung in der Synthese von Pyrazolen und Pyrazolinen wurde in Chem. Eur. J. 2016, 22, 9542-9545 "Fluoroalkyl-Substituted Diazomethanes and Their Application in a General Synthesis of Pyrazoles and Pyrazolines" offenbart. Jedoch ist auch bei diesem Verfahren die Menge des synthetisierten fluoralkylsubstituierten Diazomethans auf den Milligramm-Bereich limitiert.

Daher besteht ein Bedarf an weiteren Verbesserungen der Synthese fluoralkyl-substituierter Diazomethane. Der vorliegenden Erfindung lag daher die Aufgabe zu Grunde, ein Verfahren zur Verfügung zu stellen, dass mindestens einen der vorgenannten Nachteile des Standes der Technik überwindet. Insbesondere lag der vorliegenden Erfindung die Aufgabe zu Grunde, ein Verfahren bereit zu stellen, das für eine technische Herstellung von fluoralkyl-substituierten Diazomethanen verwendbar ist.

Diese Aufgabe wird gelöst durch ein Verfahren zur Herstellung eines fluorierten Diazoalkans, wobei es sich bei dem Verfahren um ein kontinuierliches Verfahren handelt und ein β,β-Difluoralkylamin mit einem organischen Nitrit in einem Reaktor umgesetzt wird, wobei das β,β-Difluoralkylamin und das organische Nitrit in getrennten Gefäßen vorgelegt werden.

Überraschend wurde gefunden, dass das erfindungsgemäße Verfahren die kontinuierliche Herstellung fluorierter Diazoalkane unter Erzeugung größerer Mengen erlaubt. So konnte unter Verwendung kommerziell verfügbarer β,β-Difluoralkylamine fluoriertes Diazoalkan kontinuierlich dargestellt werden und so in Gramm Mengen für die organische Synthese eingesetzt werden. Das erfindungsgemäße Verfahren erlaubt erstmals die Aufskalierung der kontinuierlichen Herstellung fluorierter Diazoalkane in den großtechnischen Maßstab und eine skalierbare Reaktionsführung. Weiterhin erlaubt die kontinuierliche Herstellung der fluorierten Diazoalkane eine direkte Weiterverwendung im Rahmen einer zweischrittigen Reaktion, wodurch das fluorierte Diazoalkan nicht akkumuliert und die Sicherheit der Synthese deutlich verbessert wird. Die hiermit verbundene Verfügbarkeit fluoralkyl-substituierter Verbindungen erlaubt ferner eine Kommerzialisierung. Die resultierenden fluoralkyl-substituierten Verbindungen, insbesondere di- oder trifluoralkyl-substituierte Heterocyclen wie Pyrazole oder Pyrazoline, besitzen großes Marktpotential insbesondere auf den Gebieten der Pharmazeutika und Agrochemikalien.

Unter dem Begriff "kontinuierliches" Verfahren wird ein Verfahren verstanden, das im Gegensatz zu einem diskontinuierlichen Chargen- oder batch-Prozess kontinuierlich, ohne Unterbrechungen abläuft. Kontinuierliche Verfahren sind insbesondere für die Herstellung oder Verarbeitung größerer Mengen vorteilhaft.

Unter dem Begriff "fluoriertes Diazoalkan" wird eine Verbindung verstanden, die neben der Diazogruppe wenigstens einen Fluorsubstituenten aufweist. Ausgehend von einem β,β-Difluoralkylamin als Edukt weisen die erhaltenen fluorierten Diazoalkane in 2,2-Stellung wenigstens zwei Fluorsubstuenten auf. Die erhältlichen fluorierten Diazoalkane können in Anlehnung an die einfachste Diazoverbindung Diazomethan auch als fluoralkyl-substituierte Diazomethane bezeichnet werden. Die erhältlichen fluorierten Diazoalkane können beispielsweise 2,2-Difluordiazoethan, oder 2,2,2-Trifluordiazoethan sein. 2,2-Difluordiazoethan kann alternativ als Difluormethyl Diazomethan oder 2,2-Difluormethyl Diazomethan bezeichnet werden. 2,2,2-Trifluordiazoethan kann alternativ Trifluormethyl Diazomethan oder 2,2,2-Trifluormethyl Diazomethan bezeichnet werden.

Unter dem Begriff "Gefäß" kann jedes Behältnis verstanden werden, das geeignet ist, insbesondere flüssige Stoffe aufzubewahren. Das Gefäß kann auch als Vorratsgefäß bezeichnet werden. Das Gefäß kann beispielsweise ein Kolben, eine Flasche, ein Tank, eine Spritze oder ein anderer in Labor oder Industrie verwendbarer Behälter sein. Erfindungsgemäß werden das β,β-Difluoralkylamin und das organische Nitrit in getrennten Gefäßen vorgelegt. Vorzugsweise wird das β,β-Difluoralkylamin mit dem organischen Nitrit in Anwesenheit einer organischen Säure, insbesondere Essigsäure, umgesetzt. Das β,β-Difluoralkylamin, das organische Nitrit und Essigsäure können in wenigstens zwei getrennten Gefäßen vorgelegt werden. In bevorzugten Ausführungformen wird das β,β-Difluoralkylamin in einem ersten Gefäß vorgelegt und das organische Nitrit und Essigsäure gemeinsam in einem zweiten Gefäß vorgelegt, oder das organische Nitrit wird in einem ersten Gefäß vorgelegt und das β,β-Difluoralkylamin und Essigsäure gemeinsam in einem zweiten Gefäß vorgelegt. Es ist hierbei insbesondere vorgesehen, dass das β,β-Difluoralkylamin und das organische Nitrit in getrennten Gefäßen vorgelegt werden. Der Katalysator Essigsäure kann dabei sowohl gemeinsam mit dem Nitrit wie auch dem Amin vorgelegt werden. Es hat sich herausgestellt, dass die Ausbeute bei beiden Maßnahmen deutlich höher ist, als wenn Amin und Nitrit gemeinsam vorgelegt werden. Es wird angenommen, dass durch beide Maßnahmen ein vorzeitiger Verbrauch des Amins und damit eine Verringerung der Ausbeute, vermieden werden kann.

In Ausführungsformen können das β,β-Difluoralkylamin, das organische Nitrit und Essigsäure im Reaktor oder vor dem Eintritt in den Reaktor vermischt werden. Hierbei können die Komponenten in einem Mischer vermischt werden. Unter dem Begriff "Mischer" wird eine Vorrichtung verstanden, die für eine Durchmischung der Reaktanden geeignet ist. Der Mischer kann beispielsweise ein T-Stück einer Schlauch- oder Rohrverbindung sein. Vorzugsweise ist der Mischer ein Mini-Mischer, insbesondere ein Mikromischer. Derartige Bauteile sind aus der Mikrofluidik bekannt und kommerziell erhältlich. Bevorzugt verwendbar sind insbesondere Mikromischer, beispielsweise aus Glas, die zusätzliche Durchmischungskanäle aufweisen können. Bevorzugte Mischer sind beispielsweise Little Things Factory, MR-Lab Series, Vici oder Upchurch Scientific PEEK Mixing Tee.

Die Reaktanden können dem Mischer oder dem Reaktor mittels einer Pumpe beispielsweise einer Dosierpumpe aus den jeweiligen Gefäßen zugeführt werden. Die Pumpe kann beispielsweise eine Spritzenpumpe oder eine HPLC-Pumpe sein. Die Pumpe kann einen Förderbereich von 10 µL bis 100 mL/min aufweisen.

Unter dem Begriff "Reaktor" wird eine Vorrichtung verstanden, in der eine chemische Reaktion abläuft. Der Reaktor ist vorzugsweise ein Mini-Reaktor, insbesondere ein MikroReaktor. Bevorzugt ist ein Durchflussreaktor, beispielsweise ein Strömungsrohr oder ein Schlauch, dem im kontinuierlichen Betrieb Edukte und Produkte besonders einfach zugeführt und abgezogen werden können. Der Reaktor kann beispielsweise aus Polytetrafluorethylen (PTFE), Fluorethylenpropylen (FEP), Perfluoralkoxy-Polymere (PFA) oder Edelstahl oder einer anderen Legierung, die üblicherweise im HPLC oder MPLC (Hochleistungs- oder Mitteldruck-Flüssigkeitschromatographie) Anwendungsbereich verwendet wird, ausgebildet sein. Entsprechende Reaktoren sind kommerziell erhältlich. Der Reaktor kann auch durch ein Rohr- oder Schlauchstück ausgebildet sein. Beispielsweise kann der Reaktor durch eine, beispielsweise an bzw. hinter einem Mischer vorgesehene, Verschlauchung ausgebildet sein. Abhängig von der apparativen Ausführung der Vorrichtung können Reaktor und Mischer durch zwei getrennte Vorrichtungen ausgebildet werden. Der Mischer muss jedoch keine vom Reaktor getrennt ausgeführte Vorrichtung sein. So können Reaktor und Mischer in anderen Ausführungsformen durch eine Vorrichtung ausgebildet sein. Beispielsweise kann der Reaktor in Ausführungsformen durch den Mischer ausgebildet sein oder diese Funktion mit erfüllen, oder umgekehrt, solange die Komponenten β,β-Difluoralkylamin, organisches Nitrit und Essigsäure zunächst vermischt werden.

Vorzugsweise ist der Reaktor an ein Rückdruckventil angeschlossen. Dieses kann das Austreten oder Entstehen von Gasen in dem Reaktor bzw. Mikroreaktor unterdrücken. Das Rückdruckventil kann beispielsweise bei einem Druck von 20 psi bis 80 psi betrieben werden.

Der Auslass des Reaktors bzw. Mikroreaktors kann mit einem weiteren Reaktionsgefäß, beispielsweise einem Kolben oder einem weiteren Reaktor insbesondere Mikroreaktor verbunden sein. Vorzugsweise ist der Auslass des Reaktors bzw. Mikroreaktors mit einem zweiten Mikroreaktor, vorzugsweise in Kombination mit einem zweiten Mikromischer, verbunden. Hierdurch kann das hergestellte fluorierte Diazoalkan in einer weiteren Reaktion mit einem Dipolarophil umgesetzt werden und zu einer als Endprodukt gewünschten fluoralkyl-substituierten Verbindung reagieren. Der zweite Reaktionsschritt erfolgt vorzugsweise in kontinuierlicher Führung, kann jedoch auch als batch-Reaktion geführt werden.

Das β,β-Difluoralkylamin und das organische Nitrit werden vorzugsweise bei erhöhter Temperatur im Reaktor zur Reaktion gebracht. In bevorzugten Ausführungsformen führt man die Reaktion des β,β-Difluoralkylamins mit dem organischen Nitrit im Reaktor bei einer Temperatur im Bereich von ≥ 40°C bis ≤ 100°C, bevorzugt im Bereich von ≥ 40°C bis ≤ 80°C, vorzugsweise bei einer Temperatur im Bereich von ≥ 55°C bis ≤ 75°C, durch. Die Reaktion läuft bereits bei Temperaturen von 40°C mit guter Ausbeute ab, jedoch kann eine Erhöhung der Temperatur zu einem schnelleren Ablauf der Reaktion sowie einer höheren Ausbeute an fluoriertem Diazoalkan führen. Ab einer Temperatur von 100°C kann die Ausbeute wieder sinken. Insbesondere in einem Temperaturbereich von ≥ 55°C bis ≤ 75°C wurde ein gutes Verhältnis von Ausbeute an fluoriertem Diazoalkan zu unverbrauchtem Amin festgestellt. Die Reaktion kann beispielsweise bei einer Temperatur von 55°C durchgeführt werden. Vorzugsweise entspricht die Temperatur im Mischer der des Reaktors. Entsprechend kann die Temperatur im Mischer im Bereich von ≥ 40°C bis ≤ 100°C, bevorzugt im Bereich von ≥ 40°C bis ≤ 80°C, vorzugsweise bei einer Temperatur im Bereich von ≥ 55°C bis ≤ 75°C, liegen.

Es wurde festgestellt, dass der Faktor Temperatur neben dem verwendeten Lösemittel und dem Verhältnis von β,β-Difluoralkylamin zu organischem Nitrit einen großen Einfluss auf die Reaktion zeigte. Weitere Parameter, die untersucht wurden, waren die Reaktionszeit sowie der Stoffmengenanteil an Essigsäure. In bevorzugten Ausführungsformen liegt die Verweilzeit der Komponenten β,β-Difluoralkylamin, organisches Nitrit und Essigsäure im Reaktor im Bereich von ≥ 30 Sekunden bis ≤ 1 Stunde, vorzugsweise im Bereich von ≥ 1 min bis ≤ 20 min, bevorzugt im Bereich von ≥ 2 min bis ≤ 10 min. Bereits bei einer Verweilzeit bzw. Reaktionsdauer von 30 Sekunden kann die Reaktion von Amin und Nitrit nahezu vollständig ablaufen, so dass die Restmenge an unverbrauchtem Amin gering ist. Dies ist insbesondere für einen nachfolgenden weiteren Reaktionsschritt vorteilhaft, da das Amin einen möglicherweise benötigten Katalysator nicht vergiftet. Abhängig von der Reaktionstemperatur kann die Reaktionsdauer angepaßt werden. Bei einer kontinuierlichen Reaktionsführung konnten bereits bei Verweilzeit im Bereich von ≥ 2 min bis ≤ 10 min bereits ein guter Stoffumsatz erzielt werden.

Das Volumen des Reaktors kann im Bereich von 200 µL bis 100 ml liegen. Anhand der Größe des verwendeten Reaktors und der geplanten Reaktionsdauer kann die Flussrate, mit der Amin, Nitrit und Essigsäure oder deren Mischungen dem Mischer aus den jeweiligen Gefäßen zugeführt werden bzw. den Reaktor durchströmen, eingestellt werden. Beispielsweise können β,β-Difluoralkylamin, organisches Nitrit und Essigsäure oder deren Mischungen dem Mischer und/oder Reaktor mit einer Flussrate im Bereich von ≥ 10 µL/min bis ≤ 100 mL/min, vorzugsweise im Bereich von ≥ 10 uL/min bis ≤ 1 mL/min, bevorzugt im Bereich von ≥ 50 uL/min bis ≤ 100 µL/min, zugeführt werden. Weiter können β,β-Difluoralkylamin, organisches Nitrit und Essigsäure den Reaktor mit einer Flussrate im Bereich von ≥ 10 uL/min bis ≤ 100 mL/min, vorzugsweise im Bereich von ≥ 10 µL/min bis ≤ 1 mL/min, bevorzugt im Bereich von ≥ 50 µL/min bis ≤ 100 µL/min, durchströmen.

Es ist für die kontinuierliche Herstellung fluorierter Diazoalkane vorteilhaft, diese im Fluid zu führen. β,β-Difluoralkylamin, organisches Nitrit und Essigsäure werden vorzugsweise in einem nicht-wässrigen Lösemittel vorgelegt. Geeignete nicht-wässrige Lösemittel sind bevorzugt ausgewählt aus Dichlormethan, Dichlorethan und Chloroform. Insbesondere Chloroform ist ein bevorzugtes Lösemittel. Es hat sich weiter als vorteilhaft herausgestellt, wenn getrocknetes Chloroform verwendet wird. So wurden bei der Verwendung getrockneten Chloroforms bessere Ausbeuten erhalten.

Die Konzentration des β,β-Difluoralkylamins im Vorratsgefäß kann im Bereich von ≥ 0,1 M bis ≤ 2 M, bevorzugt im Bereich von ≥ 0,2 M bis ≤ 0,8 M, liegen. Die Konzentration des organischen Nitrits im Vorratsgefäß kann im Bereich von ≥ 0,1 M bis ≤ 2,4 M, bevorzugt im Bereich von ≥ 0,2 M bis ≤ 1 M, liegen. Es hat sich als vorteilhaft herausgestellt, in diesen Konzentrationsbereichen zu arbeiten, da bei höheren Konzentrationen niedrigere Ausbeute erhalten wurden.

Das organische Nitrit ist vorzugsweise ein Alkylnitrit, insbesondere ein C₁-C₈-Alkylnitrit. Bevorzugt verwendbar sind Butyl-, Isobutyl-, Pentyl-, Neopentyl- oder tert-Butyl-Nitrit. Insbesondere tert-Butylnitrit ist eine günstige, kommerziell erhältliche organische Nitritquelle. Das organische Nitrit kann bezogen auf das β,β-Difluoralkylamin in stöchiometrischen Mengen oder im molaren Überschuss verwendet werden. In bevorzugten Ausführungsformen verwendet man das organische Nitrit in einem ≥ 0,5-fachen bis ≤ 2-fachen, vorzugsweise einem ≥ 1-fachen bis ≤ 1,5-fachen, bevorzugt einem ≥ 1-fachen bis ≤ 1,2-fachen, molaren Überschuss bezogen auf das β,β-Difluoralkylamin. Bei einem leichten Überschuss des organischen Nitrits konnten besonders gute Ausbeuten an fluoriertem Diazoalkan erzielt werden.

Essigsäure ist hat sich als besonders geeigneter Katalysator für die Synthese von fluorierten Diazoalkanen erwiesen. Vorzugsweise sind katalytische Mengen an Essigsäure verwendbar. In bevorzugten Ausführungsformen liegt der Stoffmengenanteil der Essigsäure im Bereich von ≥ 1 mol% bis ≤ 50 mol%, vorzugsweise im Bereich von ≥ 5 mol% bis ≤ 40 mol%, bevorzugt im Bereich von von ≥ 5 mol% bis ≤ 10 mol%, bezogen auf die Stoffmenge des β,β-Difluoralkylamins. Es konnte festgestellt werden, dass bereits ein Stoffmengenanteil an 5 mol% Essigsäure bezogen auf die Stoffmenge des β,β-Difluoralkylamins unter Verwendung von Chloroform als Lösemittel bei einer Reaktionstemperatur von 55°C zu sehr guten Ausbeuten führte. Auch konnte bei Verwendung eines Stoffmengenanteil an Essigsäure von 40 mol%, bezogen auf die Stoffmenge des β,β-Difluoralkylamins, einer Reaktionszeit von 2 Minuten bei 75°C eine Ausbeute des gewünschten Difluormethyl-diazomethans von etwa 40% erzielt werden.

Die Amingruppe des β,β-Difluoralkylamins reagiert mit dem organischen Nitrit zu einer Diazogruppe. Das β,β-Difluoralkylamin ist insofern der Präkursor für die gewünschte, vorzugsweise in 2,2-Stellung wenigstens zweifach, fluorierte Diazoverbindung. In bevorzugten Ausführungsformen weist das β,β-Difluoralkylamin die folgende allgemeinen Formel (1) auf: 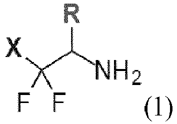 worin:
- R: ist ausgewählt aus der Gruppe umfassend H und/oder substituiertes oder unsubstituiertes Alkyl, Aryl, Arylalkyl, Cyclyl, Heteroaryl oder Heterocyclyl,
- X: ist ausgewählt aus der Gruppe umfassend H, F, Cl, CN, CO₂R', CONR', COR', SO₂R', SO₂NR'₂ und/oder substituiertes oder unsubstituiertes Alkyl, Aryl, Arylalkyl, Cyclyl, Heteroaryl oder Heterocyclyl, und
- R': ist gegebenenfalls unabhängig voneinander ausgewählt aus der Gruppe umfassend H und/oder substituiertes oder unsubstituiertes Alkyl, Aryl, Arylalkyl, Cyclyl, Heteroaryl oder Heterocyclyl.

Im Sinne der vorliegenden Erfindung sind, wenn nicht abweichend angegeben, unter dem Begriff "Heterocyclyl" mono-, bi- oder tricyclische Heterocyclylgruppen umfassend ein, zwei, drei oder vier Heteroatome ausgewählt aus der Gruppe umfassend N, O und/oder S zu verstehen. Bevorzugte Heterocyclylgruppen sind monocyclische Heterocyclylgruppen. Bevorzugte Heterocyclylgruppen umfassen ein oder zwei Heteroatome ausgewählt aus der Gruppe umfassend N, O und/oder S, vorzugsweise N. Besonders bevorzugte Heterocyclylgruppen sind ausgewählt aus der Gruppe umfassend Pyran, Furan, Piperidin und/oder Pyrrolidin.

Im Sinne der vorliegenden Erfindung sind, wenn nicht abweichend angegeben, unter dem Begriff "Heteroaryl" mono-, bi- oder tricyclische Heteroarylgruppen umfassend ein, zwei, drei oder vier Heteroatome ausgewählt aus der Gruppe umfassend N, O und/oder S zu verstehen. Bevorzugte Heteroarylgruppen sind monocyclische Heteroarylgruppen. Bevorzugte monocyclische Heteroarylgruppen weisen ein oder zwei Heteroatom(e) auf. Bevorzugte Heteroarylgruppen sind ausgewählt aus der Gruppe umfassend Pyridin, Pyrimidin, Pyrrol, Pyrazol, Imidzazol, Thiazol, Oxazol und/oder Isoxazol.

Der Begriff "C₁-₈-Alkyl" umfasst, wenn nicht anders angegeben, geradkettige oder verzweigte Alkylgruppen mit 1 bis 8 Kohlenstoffatomen. Der Begriff "Me" bezeichnet eine Methylgruppe, "Et" eine Ethylgruppe. Der Begriff "C₅₋₆-Heteroaryl" umfasst, wenn nicht anders angegeben, ein aromatisches Ringsystem mit 5 bis 6 Kohlenstoff- und Heteroatomen.

Im Sinne der vorliegenden Erfindung ist, wenn nicht abweichend angegeben, unter dem Begriff "Arylalkyl" eine Gruppe zu verstehen, die über den letztgenannten Teil gebunden ist, beispielsweise ist eine Arylalkyl-Gruppe über den Alkylteil gebunden. Bevorzugt weist die Arylgruppe 6 Kohlenstoffatome auf und der Alkylteil 1 bis 3 Kohlenstoffatome. Eine bevorzugte Arylalkyl-Gruppe ist Benzyl.

Die Alkyl-, Aryl-, Cyclyl-, Heteroaryl- und Heterocyclylgruppen können substituiert sein, beispielsweise mit einer oder mehreren Gruppen ausgewählt aus der Gruppe umfassend F, Cl, CF₃, CF₂H, CHF₂, O-C₁₋₈-Alkyl, O-C₃₋₆-Cycloalkyl, OCF₃, OCF₂H, SCF₃, SCF₂H, S-C₁₋₈-Alkyl, S-C₃₋₆-Cycloalkyl, SO-C₁₋₈-Alkyl, SO-C₃₋₆-Cycloalkyl, SO₂-C₁₋₈-Alkyl, SO₂-C₃₋₆-Cycloalkyl, CONH₂, CONHMe, CONMe₂ und/oder CO₂-C₁₋₆-Alkyl.

In Ausführungsformen des β,β-Difluoralkylamins der allgemeinen Formel (1) sind:
- R: ausgewählt aus der Gruppe umfassend H und/oder substituiertes oder unsubstituiertes C₁₋₈-Alkyl, Phenyl, Benzyl, C₃₋₆-Cyclyl, C₅₋₆-Heteroaryl oder C₃₋₆-Heterocyclyl,
- X: ausgewählt aus der Gruppe umfassend H, F, Cl, CN, CO₂R', CONR', COR', SO₂R', SO₂NR'₂ und/oder substituiertes oder unsubstituiertes C₁₋₈-Alkyl, Phenyl, Benzyl, C₃₋₆-Cyclyl, C₅₋₆-Heteroaryl oder C₃₋₆-Heterocyclyl, und
- R': gegebenenfalls unabhängig voneinander ausgewählt aus der Gruppe umfassend H und/oder substituiertes oder unsubstituiertes C₁₋₈-Alkyl, Phenyl, Benzyl, C₃₋₆-Cyclyl, C₅₋₆-Heteroaryl oder C₃₋₆-Heterocyclyl.

In bevorzugten Ausführungsformen des β,β-Difluoralkylamins gemäß Formel (1) ist X Wasserstoff, Fluor oder eine Perfluorgruppe ausgewählt aus CF₃, C₂F₅ C₃F₇ und/oder C₄F₉ oder X ist ausgewählt aus der Gruppe umfassend H, F, CF₃, CF₂H, CFH₂ und/oder C₂F₅. In weiter bevorzugten Ausführungsformen des β,β-Difluoralkylamins gemäß Formel (1) ist X = H und R = H. In weiter bevorzugten Ausführungsformen des β,β-Difluoralkylamins gemäß Formel (1) ist X = F und R = H. In ebenfalls bevorzugten Ausführungsformen des β,β-Difluoralkylamins gemäß Formel (1) ist X = F und R ist ausgewählt aus der Gruppe umfassend Methyl, Ethyl, Phenyl und/oder Phenyl substituiert mit Me, OMe, F, Cl, Br oder COOC₁₋₆-Alkyl, insbesondere COOMe, COOEt, COOtert.butyl.

Besonders bevorzugte β,β-Difluoralkylamine sind 2,2-Difluorethylamin und 2,2,2-Trifluorethylamin. Weiter bevorzugte β,β-Difluoralkylamine sind ausgewählt aus der Gruppe umfassend: 2,2,3,3,3-pentafluoropropylamin, 2,2,3,3,4,4,4-heptafluorobutylamin, 2,2,3,3,4,4,5,5,5-nonafluoropentylamin, 1,1,1-trifluoropropan-2-amin, 1,1,1-trifluorobutan-2-amin, 1,1,1-trifluoro-4-phenylbutan-2-amin, 2,2,2-trifluoro-1-phenylethan-1-amin, 2,2,2-trifluoro-1 -(4-fluorophenyl)ethan-1 -amin und/oder 2,2,2-trifluoro-1 -(4-methylphenyl)ethan-1 - amin.

Ein großer Vorteil des Verfahrens liegt darin, dass keine Extraktion, Reinigung oder Aufarbeitung des hergestellten fluorierten Diazoalkans vor der weiteren Verwendung in nachfolgenden Syntheseschritten notwendig ist. So ist das erhaltene fluorierte Diazoalkan direkt in einem nachfolgenden Reaktion zur Herstellung einer gewünschten fluorierten organischen Verbindung verwendbar. Hierzu kann das hergestellte fluorierte Diazoalkan mit einem Dipolarophil umgesetzt werden und zu der als Endprodukt gewünschten fluoralkyl-substituierten Verbindung reagieren. Der zweite Reaktionsschritt erfolgt vorzugsweise in kontinuierlicher Führung, kann jedoch auch als batch-Reaktion geführt werden.

Ein weiterer Aspekt der Erfindung betrifft die Verwendung des erfindungsgemäßen Verfahrens zur Herstellung einer fluoralkyl-substituierten Verbindung. Das Verfahren kann beispielsweise im Rahmen einer zweischrittigen Reaktion zur Herstellung von Cyclopropanen durch eine nachfolgende Cyclopropanierung oder zur Herstellung von Pyrazolen und Pyrazoline durch nachfolgendes Umsetzen des fluorierten Diazoalkans mit einem Dipolarophil in einer 1,3-dipolaren Cycloaddition verwendet werden. Bevorzugte fluoralkyl-substituierte Verbindungen sind di- oder trifluoralkyl-substituierte Pyrazole, Pyrazoline und Cyclopropane.

Ein weiterer Aspekt der Erfindung betrifft entsprechend ein Verfahren zur Herstellung einer fluoralkyl-substituierten Verbindung, insbesondere di- oder trifluoralkyl-substituierter Pyrazole, Pyrazoline und Cyclopropane, wobei das Verfahren die folgenden Schritte umfasst:
a) Herstellen eines fluorierten Diazoalkans gemäß dem oben beschriebenen Verfahren, und
b) Umsetzen des fluorierten Diazoalkans in einer Cyclopropanierung oder mit einem Dipolarophil in einer 1,3-dipolaren Cycloaddition.

Für die Beschreibung der Herstellung des fluorierten Diazoalkans gemäß Schritt a) wird auf die vorstehende Beschreibung Bezug genommen. Das Umsetzen des fluorierten Diazoalkans in einer Cyclopropanierung oder mit einem Dipolarophil in einer 1,3-dipolaren Cycloaddition gemäß Schritt b) kann entsprechend dem Fachmann bekannten Bedingungen erfolgen. Ein bevorzugtes Verfahren ist die Herstellung di- oder trifluoralkyl-substituierter Pyrazole und Pyrazoline durch eine 1,3-dipolaren Cycloaddition.

Schritt b) kann als herkömmliche, diskontinuierliche batch-Reaktion geführt werden. Das fluorierte Diazoalkan, kann hierzu, beispielsweise in einen Kolben, zu dem Dipolarophil gegeben werden. Der Vorteil einer diskontinuierlichen batch-Reaktion liegt darin, dass auf eine Isolierung des Diazoalkans verzichtet werden kann, und eine schnelle und standardisierte Versuchsdurchführung für Schritt b) für Umsetzungen im kleinen Maßstab zur Verfügung gestellt werden kann. Bereits in einem diskontinuierlichen Ansatz für Schritt b) konnten Ausbeuten von mehr als einem Gramm an Difluormethyl-substituiertem Pyrazolin erhalten werden.

Alternativ kann Schritt b) in kontinuierlicher Führung erfolgen. Hierzu kann der Auslass des ersten für Schritt a) verwendeten Reaktors mit einem weiteren Reaktor insbesondere Mikroreaktor verbunden sein. Die Dimensionierung des zweiten Reaktors ist unabhängig von der des ersten Reaktors und im Rahmen der gewünschten Reaktionsführung wählbar. Für eine kontinuierliche Reaktion kann in Schritt a) ein erster Mikroreaktor verwendet werden und in Schritt b) ein zweiter Mikroreaktor. Der große Vorteil einer kontinuierlichen Reaktion liegt darin, dass eine erhöhte Sicherheit der Herstellung und des Arbeitens mit Diazomethanen zur Verfügung gestellt werden kann. Ein besonderer Vorteil liegt weiterhin in der Skalierbarkeit einer kontinuierlichen Herstellung. Es konnte gezeigt werden, dass sowohl die diskontinuierliche wie auch die kontinuierliche Reaktionsführung in Schritt b) zu sehr guten Ausbeuten an di- und trifluoralkyl-substituierten Pyrazolen und Pyrazolinen führte.

Vorzugsweise ist der Auslass des ersten Reaktors bzw. Mikroreaktors mit einem zweiten Mischer bzw. Mikromischer verbunden. In den zweiten Mischer kann ebenfalls das Dipolarophil eingespeist werden.

Diazoalkan und Dipolarophil können in einer diskontinuierlichen wie auch in einer kontinuierlichen Reaktionsführung bei einer Temperatur im Bereich von ≥ 0°C bis ≤ 75 °C und/oder für eine Reaktionszeit im Bereich von ≥ 10 min bis ≤ 14 h, gegebenfalls unter Rühren und/oder Zusetzen eines Katalysators, umgesetzt werden. Die Cyclopropanierung findet bevorzugt zwischen für eine Reaktionszeit im Bereich von ≥ 10 min bis ≤ 6 Stunden und/oder bei einer Temperatur im Bereich von ≥ 0°C bis 40 °C in einer batch Reaktion in Anwesenheit eines geeigneten Katalysators statt.

Vorzugsweise ist das Dipolarophil in einem Lösemittel gelöst. Geeignete nicht-wässrige Lösemittel sind bevorzugt ausgewählt aus Dichlormethan, Dichlorethan und Chloroform. Das Lösemittel kann dem in Schritt a) verwendeten Lösemittel entsprechen. Insbesondere Chloroform ist ein bevorzugtes Lösemittel für die Herstellung di- oder trifluoralkyl-substituierter Cyclopropane. Für die Herstellung aryl- und alkyl-sulfonsubstituierter Pyrazoline hat sich Dichlormethan als bevorzugtes Lösemittel erwiesen.

Bevorzugte di- oder trifluoralkyl-substituierte Pyrazole und Pyrazoline sind ausgewählt aus der Gruppe umfassend:
Methyl 3-(difluoromethyl)-1*H*-pyrazol-5-carboxylat,
Ethyl 3-(difluoromethyl)-1*H*-pyrazol-5-carboxylat,
Tert-butyl 3-(difluoromethyl)-1*H*-pyrazol-5-carboxylat,
5-Benzyl-3-(difluoromethyl)-1,6*a*-dihydropyrrolo[3,4-*c*]pyrazol-4,6(3*aH*,5*H*)-dion,
1-(3-(Difluoromethyl)-1*H*-pyrazol-5-yl)ethanon,
Dimethyl 3-(difluoromethyl)-1*H*-pyrazol-4,5-dicarboxylat,
Tert-butyl 3-(difluoromethyl)-4,5-dihydro-1*H*-pyrazol-5-carboxylat,
1-(3-(difluoromethyl)-4,5-dihydro-1*H*-pyrazol-5-yl)ethanon,
Tert-butyl 3-(trifluoromethyl)-1*H*-pyrazol-5-carboxylat,
Methyl 3-(trifluoromethyl)-1*H*-pyrazol-5-carboxylat,
Ethyl 3-(trifluoromethyl)-1*H*-pyrazol-5-carboxylat,
1-(3-(Trifluoromethyl)-1*H*-pyrazol-5-yl)ethanon,
Dimethyl 3-(trifluoromethyl)-1*H*-pyrazol-4,5-dicarboxylat,
1-(3-(trifluoromethyl)-4,5-dihydro-1*H*-pyrazol-5-yl)ethanon,
Tert-butyl 3-(trifluoromethyl)-4,5-dihydro-1*H*-pyrazol-5-carboxylat,
Ethyl 5-(difluoromethyl)-4-(trifluoromethyl)-4,5-dihydro-1H-pyrazole-3-carboxylat und/oder
Ethyl 3-(difluoromethyl)-4-(trifluoromethyl)-1H-pyrazole-5-carboxylat.

Besonders bevorzugte di- oder trifluoralkyl-substituierte Pyrazole und Pyrazoline sind ausgewählt aus der Gruppe umfassend Methyl 3-(difluoromethyl)-1*H*-pyrazol-5-carboxylat, Ethyl 3-(difluoromethyl)-1*H*-pyrazol-5-carboxylat und/oder Tert-butyl 3-(difluoromethyl)-1*H*-pyrazol-5-carboxylat. Es kann in vorteilhafter Weise ein Zugang zu difluormethylcarboxylsubstituierten Pyrazolen zur Verfügung gestellt werden. Das Verfahren stellt in vorteilhafter Weise eine wirtschaftliche und skalierbare Methode für die direkte Synthese fluoralkyl-substituierter Pyrazole und Pyrazoline ausgehend von Propiolsäureestern als Dipolarophil zur Verfügung.

Bevorzugte di- oder trifluoralkyl-sulfonyl substituierte Pyrazole und Pyrazoline sind ausgewählt aus der Gruppe umfassend:
3-(Methylsulfonyl)-5-(trifluoromethyl)-4,5-dihydro-1*H*-pyrazol,
3-(Ethylsulfonyl)-5-(trifluoromethyl)-4,5-dihydro-1*H*-pyrazol,
3-(Benzylsulfonyl)-5-(trifluoromethyl)-4,5-dihydro-1*H*-pyrazol,
3-(Phenylsulfonyl)-5-(trifluoromethyl)-4,5-dihydro-1*H*-pyrazol,
3-((4-Methylphenyl)sulfonyl)-5-(trifluoromethyl)-4,5-dihydro-1*H*-pyrazol,
3-((4-Fluorophenyl)sulfonyl)-5-(trifluoromethyl)-4,5-dihydro-1*H*-pyrazol,
3-((4-Chlorophenyl)sulfonyl)-5-(trifluoromethyl)-4,5-dihydro-1*H*-pyrazol,
3-((4-Bromophenyl)sulfonyl)-5-(trifluoromethyl)-4,5-dihydro-1*H*-pyrazol,
3-((4-Methoxyphenyl)sulfonyl)-5-(trifluoromethyl)-4,5-dihydro-1*H*-pyrazol,
3-((3-Methylphenyl)sulfonyl)-5-(trifluoromethyl)-4,5-dihydro-1*H*-pyrazol,
3-((2-Methylphenyl)sulfonyl)-5-(trifluoromethyl)-4,5-dihydro-1*H*-pyrazol,
3-(Methylsulfonyl)-5-(difluoromethyl)-4,5-dihydro-1*H*-pyrazol,
3-(Ethylsulfonyl)-5-(difluoromethyl)-4,5-dihydro-1*H*-pyrazol,
3-(Benzylsulfonyl)-5-(difluoromethyl)-4,5-dihydro-1*H*-pyrazol,
3-(Phenylsulfonyl)-5-(difluoromethyl)-4,5-dihydro-1*H*-pyrazol,
3-((4-Methylphenyl)sulfonyl)-5-(difluoromethyl)-4,5-dihydro-1*H*-pyrazol,
3-((4-Fluorophenyl)sulfonyl)-5-(difluoromethyl)-4,5-dihydro-1*H*-pyrazol,
3-((4-Chlorophenyl)sulfonyl)-5-(difluoromethyl)-4,5-dihydro-1*H*-pyrazol,
3-((4-Bromophenyl)sulfonyl)-5-(difluoromethyl)-4,5-dihydro-1*H*-pyrazol,
3-((4-Methoxyphenyl)sulfonyl)-5-(difluoromethyl)-4,5-dihydro-1*H*-pyrazol,
3-((3-Methylphenyl)sulfonyl)-5-(difluoromethyl)-4,5-dihydro-1*H*-pyrazol,
3-((2-Methylphenyl)sulfonyl)-5-(difluoromethyl)-4,5-dihydro-1*H*-pyrazol,
3-(Methylsulfonyl)-5-(methyl)-5-(trifluoromethyl)-4,5-dihydro-1*H*-pyrazol,
3-(Phenylsulfonyl)-5-(methyl)-5-(trifluoromethyl)-4,5-dihydro-1*H*-pyrazol,
3-(Methylsulfonyl)-5-phenethyl-5-(trifluoromethyl)-4,5-dihydro-1*H*-pyrazol,
3-(Methylsulfonyl)-5-(phenyl)-5-(trifluoromethyl)-4,5-dihydro-1*H*-pyrazol,
3-(Methylsulfonyl)-5-(4-Methylphenyl)-5-(trifluoromethyl)-4,5-dihydro-1*H*-pyrazol und/oder
3-(Methylsulfonyl)-5-(4-Fluorophenyl)-5-(trifluoromethyl)-4,5-dihydro-1*H*-pyrazol.

Derartige fluoralkyl-substituierte, und insbesondere sulfon- und fluoralkyl-substituierte, Pyrazole und Pyrazoline sind für die pharmazeutische und agrochemische Industrie von hoher Bedeutung, da diese eine erhöhte Lipophilie und metabolische Stabilität aufweisen können. Das Verfahren stellt in vorteilhafter Weise auch eine wirtschaftliche und skalierbare Methode für die direkte Synthese fluoralkyl-substituierter sowie sulfon- und fluoralkyl-substituierter Pyrazole und Pyrazoline zur Verfügung. So konnte gezeigt werden, dass eine große Bandbreite an verschiedenen aromatischen Verbindungen und aliphatischen Vinylsulfonen zu den entsprechenden trifluormethyl- und sulfon-substituierten Pyrazolinen umgesetzt werden konnten. Zudem wurden diese in hervorragender Ausbeute erhalten.

In bevorzugten Ausführungsformen weist das Dipolarophil die folgende allgemeinen Formel (2) oder (3) auf: 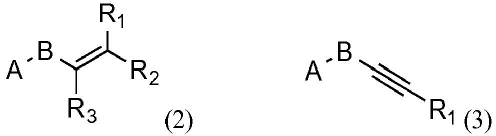 worin:
- A: ist ausgewählt aus der Gruppe umfassend substituiertes oder unsubstituiertes Alkyl, Aryl, Arylalkyl, Cyclyl, Heteroaryl, Heterocyclyl, Alkoxy, Aryloxy, NR"₂ und/oder NHR",
- B: ist ausgewählt aus der Gruppe umfassend CO und/oder SO₂,
- R₁, R₂, R₃: sind jeweils unabhängig voneinander ausgewählt aus der Gruppe umfassend H, F, Cl, C₁₋₃-Alkyl, CF₃, CF₂H, CFH₂, Phenyl, COR'" und/oder SO₂R"',
- R": ist gegebenenfalls unabhängig voneinander ausgewählt aus der Gruppe umfassend C₁₋₈-Alkyl, Phenyl und/oder C₅₋₆-Heteroaryl, und
- R'": ist ausgewählt aus der Gruppe umfassend H und/oder substituiertes oder unsubstituiertes Alkyl, Aryl, Arylalkyl, Cyclyl, Heteroaryl oder Heterocyclyl,
wobei im Fall des Dipolarophils der Formel (2) A und R₁ gemeinsam einen 5-, 6- oder 7-gliedrigen Ring ausbilden können, der als Keton, Lacton, Lactam, Sulfon, Sulfonsäurester, Sultam, Anhydrid oder Imid vorliegt.

In weiter bevorzugten Ausführungsformen des Dipolarophils der allgemeinen Formeln (2) und (3) sind:
- A: ist ausgewählt aus der Gruppe umfassend C₁₋₈-Alkyl, Phenyl, Benzyl, C₃₋₆-Cyclyl, C₆-Heteroaryl, C₃₋₆-Heterocyclyl, C₁₋₈-Alkoxy, Phenyloxy, NR"₂ und/oder NHR";
- B: ist ausgewählt aus der Gruppe umfassend CO und/oder SO₂,
- R₁, R₂, R₃: sind jeweils unabhängig voneinander ausgewählt aus der Gruppe umfassend H, F, Cl, C₁₋₃-Alkyl, CF₃, CF₂H, CFH₂, Phenyl, COR', und/oder SO₂R',
- R": ist gegebenenfalls unabhängig voneinander ausgewählt aus der Gruppe umfassend C₁₋₈-Alkyl, Phenyl, Benzyl, und/oder C₅₋₆-Heteroaryl, und
- R'": ist ausgewählt aus der Gruppe umfassend H und/oder substituiertes oder unsubstituiertes C₁₋₈-Alkyl, Phenyl, Benzyl, C₃₋₆-Cyclyl, C₅₋₆-Heteroaryl oder C₃₋₆-Heterocyclyl,
wobei im Fall des Dipolarophils der Formel (2) A und R₁ gemeinsam einen 5-, 6- oder 7-gliedrigen Ring ausbilden können, der als Keton, Lacton, Lactam, Sulfon, Sulfonsäurester, Sultam, Anhydrid oder Imid vorliegt.

Bevorzugte Dipolarophile sind ausgewählt aus der Gruppe umfassend Methylpropiolat, Ethylpropiolat, tert-Butyl-Propiolat, Methylacrylat, Ethylacrylat und/oder tert-Butyl-Acrylat.

Ein weiterer Vorteil ergibt sich daraus, dass das Verfahren für die Herstellung von fluorierten Cyclopropanen geeignet ist. In diesen Ausführungsformen ist das Dipolarophil vorzugsweise ausgewählt aus unsubstituiertem Styrol oder substituiertem Styrol wie Inden. Styrol wird nach der IUPAC-Nomenklatur als Phenylethen bezeichnet. Styrol kann beispielsweise mit Substituenten ausgewählt aus F, Cl, Br, C₁₋₈-Alkyl, insbesondere Methyl oder tert.-Butyl, substituiert sein. So konnte mittels des erfindungsgemäßen Verfahrens erstmalig eine effiziente, einfache und wirtschaftliche Synthese von Difluormethyl-substituierten Cyclopropanen ausgehend von kommerziell erhältlichen Olefinen und Aminen ermöglicht werden. Für die Herstellung der di- oder trifluoralkyl-substituierten Cyclopropane ist das β,β-Difluoralkylamin vorzugsweise Difluorethylamin.

Insgesamt kann ein Verfahren zur Verfügung gestellt werden, das unter Verwendung kommerziell verfügbarer Edukte eine Herstellung fluoralkyl-substituierter Diazomethane unter industriell skalierbaren Bedingungen in guter Ausbeute und erhöhter Sicherheit erlaubt. Das Verfahren erlaubt die Aufskalierung dieser Reaktion in den technischen Maßstab. Weiterhin gestattet die kontinuierliche Verfügbarkeit der fluoralkyl-substituierten Diazomethane eine zweischrittige Reaktionsführung für die Synthese fluoralkyl-substituierter, und insbesondere di- oder trifluoralkyl-substituierter bzw. sulfon- und fluoralkyl-substituierter, Pyrazole und Pyrazoline in hohen Ausbeuten. Hierdurch kann eine Weiterentwicklung und Kommerzialisierung dieser Verbindungen zur Verfügung gestellt werden.

Beispiele und Figuren, die der Veranschaulichung der vorliegenden Erfindung dienen, sind nachstehend angegeben.

Hierbei zeigt:
- Figur 1: die Herstellung eines fluorierten Diazoalkans am Beispiel von Difluorethylamin gemäß einer Ausführungsform des erfindungsgemäßen Verfahrens.
- Figur 2: die Herstellung einer di- oder trifluoralkyl-substituierten Verbindung unter Herstellung eines fluorierten Diazoalkans und nachfolgender Umsetzung des fluorierten Diazoalkans mit einem Dipolarophil.
- Figur 3: die Ausbeuten bei der Herstellung von Difluormethyldiazomethan gemäß Vergleichsbeispiel 72 bei Raumtemperatur in Figur 3a) und 55°C in Figur 3b).
- Figur 4: die Ausbeuten an Difluormethyldiazomethan gemäß Beispiel 73 bis 79.

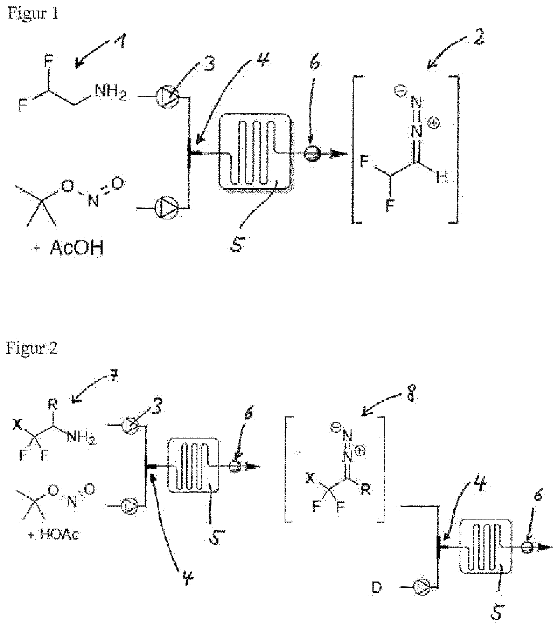

Die Figur 1 zeigt, dass durch Umsetzen von 2,2-Difluorethylamin 1 mit tert-Butylnitrit unter Verwendung von Essigsäure (AcOH) 2,2-Difluormethyldiazomethan 2 gebildet wird. Erfindungsgemäß erfolgt die Herstellung des 2,2-Difluormethyldiazomethans 2 in einem kontinuierlichen Verfahren in einem Reaktor 5. In der gezeigten Ausführungsform ist Difluorethylamin 1 in einem ersten Gefäß vorgelegt, und tert-Butylnitrit und Essigsäure sind gemeinsam in einem zweiten Gefäß vorgelegt. Vor dem Eintritt in den Reaktor 5 werden 2,2-Difluorethylamin 1, tert-Butylnitrit und Essigsäure in dem Mischer 4 vermischt. Der Mischer 4 ist gemäß Figur 1 als T-Stück ausgeführt. 2,2-Difluorethylamin 1, tert-Butylnitrit und Essigsäure werden dem Mischer 4 jeweils mittels einer Pumpe 3 zugeführt. An den Reaktor 5 ist ein Rückdruckventil 6 angeschlossen.

Die Figur 2 zeigt, die Umsetzung eines β,β-Difluoralkylamins gemäß der Formel (1) 7 mit tert-Butylnitrit unter Verwendung von Essigsäure (AcOH) zu einem in 2,2-Stellung fluorierten Diazoalkan 8. Erfindungsgemäß erfolgt die Herstellung des fluorierten Diazoalkans 8 in einem kontinuierlichen Verfahren in einem Reaktor 5. In der gezeigten Ausführungsform ist das β,β-Difluoralkylamin 7 in einem ersten Gefäß vorgelegt, und tert-Butylnitrit und Essigsäure sind gemeinsam in einem zweiten Gefäß vorgelegt. Das β,β-Difluoralkylamin 7, tert-Butylnitrit und Essigsäure werden mittels einer Pumpe 3 einem als T-Stück ausgeführten Mischer 4 zugeführt, vermischt und gelangen in den Reaktor 5. Am Reaktor 5 ist ein Rückdruckventil 6 angeschlossen.

Das fluorierte Diazoalkan 8 wird einem zweiten als T-Stück ausgeführten Mischer 4 zugeführt. Diesem wird mittels einer weiteren Pumpe 3 ebenfalls ein Dipolarophil D zugeführt. Das fluorierte Diazoalkan 8 und das Dipolarophil D werden im zweiten Mischer 4 vermischt und in einen zweiten Reaktor 5 geleitet. Am zweiten Reaktor 5 ist wiederum ein weiteres Rückdruckventil 6 angeschlossen. Das fluorierte Diazoalkan 8 und das Dipolarophil D werden im zweiten Reaktor 5 in einer 1,3-dipolaren Cycloaddition zu einer fluoralkyl-substituierten Verbindung.

### Material:

Chloroform wurde vor der Verwendung über Calciumhydrid getrocknet.

### Beispiel 1

### Kontinuierliche Herstellung von Difluormethyl-diazomethan

Eine Lösung von Difluorethylamin (0,2 M, 4 Äq) wurde in Chloroform in einer Spritze vorgelegt. In einer weiteren Spritze wurde eine Mischung von 40 mol% Essigsäure (1,6 Äq) und tert.-Butylnitrit (0,24 M, 4,8 Äq) in Chloroform bei Raumtemperatur (20±2°C) vorgelegt. Beide Lösungen wurden mittels einer Spritzenpumpe (Chemyx Fusion V710) mit einer konstanten Flussrate von jeweils 50 µL/min in einen Mikromixer (Little Things Factory, MR-LAB Type MST, Volumen 200 µL) eingebracht. Dieser diente auch als Mikroreaktor. An den Reaktor war ein Rückdruckventil (IDEX back pressure assembly) angeschlossen, das einen Druck von 40 psi aufbrachte. Der Mikromixer/Mikroreaktor wurde auf eine Temperatur von 75°C erhitzt. Die Mischung der Reaktanden hatte eine Verweilzeit von 2 Minuten im Reaktor.

Eine Probe der Reaktionslösung wurde am Auslass des Reaktors entnommen und mittels NMR untersucht. Difluormethyl-diazomethan wurde in einer Ausbeute von etwa 40% erhalten.

Die Umsetzung von Difluorethylamin (4 Äq) mit tert.-Butylnitrit (4,8 Äq) wurde unter Verwendung verschiedener Anteile an Essigsäure, Temperaturen und Verweilzeiten im Reaktor wiederholt. Proben der Reaktionslösung wurden jeweils am Auslass des Reaktors entnommen und mittels NMR untersucht. Die Bedingungen und Ausbeuten der einzelnen Umsetzungen sind in der folgenden Tabelle 1 zusammengefasst.

**Tabelle 1: Kontinuierliche Umsetzung von Difluorethylamin mit (1,2 Äq) tert.-Butylnitrit zu Difluormethyl-diazomethan**

| Beispiel | Reaktionsbedingungen | | | Ausbeute, % |
|---|---|---|---|---|
| | Essigsäure, mol% | Temperatur, °C | Verweilzeit im Reaktor, min | |
| 2 | 20 mol-% | 75 °C | 2 min | 30% |
| 3 | 10 mol-% | 55 °C | 2 min | 11% |
| 4 | 10 mol-% | 75 °C | 8 min | 15% |
| 5 | 40 mol-% | 90 °C | 2 min | 27% |
| 6 | 10 mol-% | 75 °C | 2 min | 29% |
| 7 | 10 mol-% | 75 °C | 4 min | 23% |
| 8* | 5 mol-% | 55 °C | 10 min | 26% |

| | | | | |
|---|---|---|---|---|
| *an den Mikromischer wurde zusätzlich ein Mikroreaktor mit einem Volumen von 0,8 mL angefügt. Das Gesamtvolumen des Mikromischers und -reaktors betrug 1 mL. | | | | |

Es zeigte sich, dass eine Temperatur von 55°C bis 90°C, eine Verweilzeit der Komponenten im Mischer / Reaktor von 2 bis 10 Minuten und ein Stoffmengenanteil an Essigsäure von 10 mol% bis 40 mol% gute Ausbeuten an Difluormethyl-diazomethan erzielte.

### Beispiel 9

### Verwendung des Difluormethyl-diazomethans in Cyclopropanierungen

Difluormethyl-diazomethan wurde wie in Beispiel 1 beschrieben hergestellt. Die Reaktionslösung aus dem Reaktor wurde unter Argonatmosphäre in einen 50 ml-Reaktionskolben, der 0,4 mmol Styrol (1Äq) und als Katalysator 5 mol-% Rhodium (II) (Rh₂esp₂) in 4 mL CHCl₃ enthielt, übergeleitet und bei Raumtemperatur (20±2°C) für 4 Stunden gerührt. Anschließend wurde das Chloroform im Vakuum entfernt und der Rückstand säulenchromatographisch aufgereinigt. 1-Phenyl-2-difluormethyl-cyclopropan wurde in einer Ausbeute von 67% erhalten.

Die Umsetzung der Reaktionslösung aus dem Reaktor wurde mit verschiedenen Styrolderivaten wiederholt. Die eingesetzten Styrolderivate, erhaltenen difluormethyl-substituierten Cyclopropane und jeweiligen Ausbeuten sind in der folgenden Tabelle 2 zusammengefasst.

**Tabelle 2: Umsetzung von Difluormethyl-diazomethan mit Styrolderivaten zu difluormethyl-substituierten Cyclopropanen**

| Beispiel | Styrol-Edukt | Cyclopropan | Ausbeute, % |
|---|---|---|---|
| 10 | 4-Methyl Styrol | 1-(4-Methylphenyl)-2-difluormethyl-cyclopropan | 68 |
| 11 | 4-tert. Butyl Styrol | 1-(4-tert-Butylphenyl)-2-difluormethyl-cyclopropan | 57 |
| 12 | 4-Fluor Styrol | 1-(4-Fluorophenyl)-2-difluormethyl-cyclopropan | 42 |
| 13 | 4-Chlor Styrol | 1-(4-Chlorophenyl)-2-difluormethyl-cyclopropan | 56 |
| 14 | 4-Brom Styrol | 1-(4-Bromophenyl)-2-difluormethyl-cyclopropan | 59 |
| 15 | 3-Methyl Styrol | 1-(3-Methylphenyl)-2-difluormethyl-cyclopropan | 60 |
| 16 | 3-Fluor Styrol | 1-(3-Fluorophenyl)-2-difluormethyl-cyclopropan | 52 |
| 17 | 3-Chlor Styrol | 1-(3-Chlorophenyl)-2-difluormethyl-cyclopropan | 34 |
| 18 | 2-Fluor Styrol | 1-(2-Fluorophenyl)-2-difluormethyl-cyclopropan | 49 |
| 19 | 2-Vinyl Naphthtalin | 1-(2-Naphthalenyl)-2-difluormethyl-cyclopropan | 32 |
| 20 | alpha-Methyl Styrol | (2-(difluoromethyl)-1-methylcyclopropyl)benzen | 63 |
| 21 | trans-beta Methyl Styrol | (2-(difluoromethyl)-3-methylcyclopropyl)benzen | 45 |
| 22 | Inden | 1-(difluoromethyl)-1,1a,6,6a-tetrahydro-cyclopropa[*a*]indan | 50 |
| 23 | Dihydronaphthalin | 1-(difluoromethyl)-1a,2,3,7b-tetrahydro-1H-cyclopropa[a]naphthalin | 49 |
| 24 | 4-Vinyl Pyridin | 1-(4-pyridyl)-2-difluormethyl-cyclopropan | < 10 |

Wie man der Tabelle 2 entnimmt, erlaubt das Verfahren eine effiziente Synthese difluormethyl-substituierter Cyclopropane mit guter Ausbeute. Wesentlich ist hierfür die kontinuierliche Herstellung des Difluormethyl-diazomethans in guter Ausbeute im ersten Schritt. Wie man der Tabelle 2 weiter entnimmt, ergab die Verwendung von Heterocyclen nur geringe Ausbeuten des gewünschten Cyclopropans.

### Beispiel 25

### Herstellung von Methyl 3-(difluoromethyl)-1H-pyrazol-5-carboxylat

### a) Herstellung des fluorierten Diazoalkans

Es wurden zwei Stammlösungen vorbereitet. 2,2-Difluoroethylamin (0,1 M, 2 Äq) wurde in 9 mL CHCl₃ gelöst. tBuONO (0,24 M, 2,4 Äq) und 5 mol% Essigsäure (0,08 M, 0,1 Äq) wurden ebenfalls in 9 mL CHCl₃ gelöst. Beide Lösungen wurden jeweils in einer Spritze vorgelegt und mittels Spritzenpumpe mit einer Flussrate von je 50 µL/min über einen Mikromischer (Little Things Factory, MR Lab Typ MST) mit einem Volumen von 0,2 mL und einen Mikroreaktor (CS Chromatographie PTFE tubing, Art Nr. 590515, PTFE Schlauch, ID = 0,8 mm, Länge 1,6 m) mit einem Volumen von 0,8 mL addiert. Der Mikromischer und der Mikroreaktor wurden auf 55 °C erwärmt. Die Mischung der Reaktanden hatte eine Verweilzeit von 10 Minuten im Mikroreaktor.

### b) Umsetzen des fluorierten Diazoalkans mit einem Dipolarophil in einer 1,3-dipolaren Cycloaddition

Das Schlauchende des Reaktors wurde in einen Kolben geleitet, in dem 1 Äq Ethylpropiolat (0,5 mmol), gelöst in 6 mL Chloroform, vorgelegt wurde. Nach beendeter Zugabe wurde die Reaktionslösung über Nacht bei Raumtemperatur (20±2°C) gerührt. Das Lösemittel wurde im Vakuum entfernt, der Rückstand säulenchromatographisch aufgereinigt und und Methyl 3-(difluoromethyl)-1*H*-pyrazol-5-carboxylat erhalten.

Die Umsetzung wurde mit verschiedenen Dipolarophilen wiederholt. Die erhaltenen di- und trifluormethyl-substituierten Pyrazole, Dipolarophile, und jeweiligen Fluoralkylgruppen sind in der folgenden Tabelle 3 zusammengefasst.

**Tabelle 3: Herstellung verschiedener di- und trifluormethyl-substituierter Pyrazole und Pyrazoline**

| Beispiel | di- oder trifluormethyl-substituiertes Pyrazol | Dipolarophil | Fluoralkylgruppe |
|---|---|---|---|
| 26 | Ethyl 3-(difluoromethyl)-1*H-*pyrazol-5-carboxylat | Propiolat | CF₂H |
| 27 | Tert-butyl 3-(difluoromethyl)-1*H-*pyrazol-5-carboxylat | Propiolat | CF₂H |
| 28 | 5-Benzyl-3-(difluoromethyl)-1,6*a-*dihydropyrrolo[3,4-*c*]pyrazol-4,6(3*aH*,5*H*)-dion | Maleimid | CF₂H |
| 29 | 1-(3-(Difluoromethyl)-1*H*-pyrazol-5-yl)ethanon | Alkinylketon | CF₂H |
| 30 | Dimethyl 3-(difluoromethyl)-1*H-*pyrazol-4,5-dicarboxylat | Alkinyl Diester | CF₂H |
| 31 | Tert-butyl 3-(difluoromethyl)-4,5-dihydro-1*H*-pyrazol-5-carboxylat | Acrylester | CF₂H |
| 32 | 1-(3-(difluoromethyl)-4,5-dihydro-1*H*-pyrazol-5-yl)ethanon | Vinyl keton | CF₂H |
| 33 | Tert-butyl 3-(trifluoromethyl)-1*H-*pyrazol-5-carboxylat | Propiolat | CF₃ |
| 34 | Methyl 3-(trifluoromethyl)-1*H-*pyrazol-5-carboxylat | Propiolat | CF₃ |
| 35 | Ethyl 3-(trifluoromethyl)-1*H-*pyrazol-5-carboxylat | Propiolat | CF₃ |
| 36 | 1-(3-(Trifluoromethyl)-1*H*-pyrazol-5-yl)ethanon | Alkinylketon | CF₃ |
| 37 | Dimethyl 3-(trifluoromethyl)-1*H-*pyrazol-4,5-dicarboxylat | Alkinyl Diester | CF₃ |
| 38 | 1-(3-(trifluoromethyl)-4,5-dihydro-1*H*-pyrazol-5-yl)ethanon | Vinyl keton | CF₃ |
| 39 | Tert-butyl 3-(trifluoromethyl)-4,5-dihydro-1*H*-pyrazol-5-carboxylat | Acrylester | CF₃ |

Wie man der Tabelle 3 entnimmt, erlaubt das Verfahren eine effiziente Synthese fluoroalkylsubstituierten Pyrazolen und Pyrazolinen. Wesentlich ist hierfür die kontinuierliche Herstellung der fluorierten Diazoverbindung in guter Ausbeute im ersten Schritt.

### Beispiel 40

### Herstellung von 3-(Phenylsulfonyl)-5-(difluoromethyl)-4,5-dihydro-1H-pyrazol

### a) Herstellung des fluorierten Diazoalkans

Es wurden zwei Stammlösungen vorbereitet. 2,2-Difluoroethylamin (0,2 M, 4 Äq) wurde in CHCl₃ gelöst. Tert-Butylnitrit (0,24 M, 4,8 Äq) und 5 mol% Essigsäure (0,01 M, 0,2 Äq) wurden ebenfalls in CHCl₃ gelöst. Beide Lösungen wurden jeweils in einer Spritze vorgelegt und mittels Spritzenpumpe mit einer Flussrate von je 50 µL/min über einen Mikromischer (Little Things Factory, MR Lab Typ MST) mit einem Volumen von 0,2 mL und einen Mikroreaktor (CS Chromatographie PTFE tubing, Art Nr. 590515, PTFE Schlauch, ID = 0,8 mm, Länge 1,6 m) mit einem Volumen von 0,8 mL addiert. Der Mikromischer und der Mikroreaktor wurden auf 55°C erwärmt. Die Mischung der Reaktanden hatte eine Verweilzeit von 10 Minuten im Mikroreaktor.

### b) Umsetzen des fluorierten Diazoalkans mit einem Dipolarophil in einer 1,3-dipolaren Cycloaddition

Das Schlauchende des Reaktors wurde in einen Kolben geleitet, in dem 1 Äq Phenyl Vinylsufon (0,5 mmol), gelöst in Chloroform, vorgelegt wurde. Nach beendeter Zugabe wurde die Reaktionslösung für 24 Stunden bei Raumtemperatur (20±2°C) gerührt. Das Lösemittel wurde im Vakuum entfernt, der Rückstand säulenchromatographisch aufgereinigt und 3-(phenylsulfonyl)-5-(difluoromethyl)-4,5-dihydro-1*H*-pyrazol erhalten.

Die Umsetzung wurde mit verschiedenen Vinylsulfonen wiederholt. Die erhaltenen di- und trifluormethyl-substituierten Pyrazole, eingesetzten Vinylsulfone, und jeweiligen Fluoralkylgruppen sind in der folgenden Tabelle 4 zusammengefasst.

**Tabelle 4: Herstellung verschiedener di- und trifluormethyl-sulfonyl substituierter Pyrazole und Pyrazoline**

| Beispiel | di- oder trifluormethyl-sulfonyl substituiertes Pyrazol | Dipolarophil | Fluoralkylgruppe |
|---|---|---|---|
| 41 | 3-(Methylsulfonyl)-5-(trifluoromethyl)-4,5-dihydro-1*H-*pyrazol | Vinylsulfon | CF₃ |
| 42 | 3-(Ethylsulfonyl)-5-(trifluoromethyl)-4,5-dihydro-1*H*-pyrazol | Vinylsulfon | CF₃ |
| 43 | 3-(Benzylsulfonyl)-5-(trifluoromethyl)-4,5-dihydro-1*H-*pyrazol | Vinylsulfon | CF₃ |
| 44 | 3-(Phenylsulfonyl)-5-(trifluoromethyl)-4,5-dihydro-1*H-*pyrazol | Vinylsulfon | CF₃ |
| 45 | 3-((4-Methylphenyl)sulfonyl)-5-(trifluoromethyl)-4,5-dihydro-1*H*-pyrazol | Vinylsulfon | CF₃ |
| 46 | 3-((4-Fluorophenyl)sulfonyl)-5-(trifluoromethyl)-4,5-dihydro-1*H*-pyrazol | Vinylsulfon | CF₃ |
| 47 | 3-((4-Chlorophenyl)sulfonyl)-5-(trifluoromethyl)-4,5-dihydro-1*H*-pyrazol | Vinylsulfon | CF₃ |
| 48 | 3-((4-Bromophenyl)sulfonyl)-5-(trifluoromethyl)-4,5-dihydro-1*H*-pyrazol | Vinylsulfon | CF₃ |
| 49 | 3-((4-Methoxyphenyl)sulfonyl)-5-(trifluoromethyl)-4,5-dihydro-1*H*-pyrazol | Vinylsulfon | CF₃ |
| 50 | 3-((3-Methylphenyl)sulfonyl)-5-(trifluoromethyl)-4,5-dihydro-1*H*-pyrazol | Vinylsulfon | CF₃ |
| 51 | 3-((2-Methylphenyl)sulfonyl)-5-(trifluoromethyl)-4,5-dihydro-1*H*-pyrazol | Vinylsulfon | CF₃ |
| 52 | 3-(Methylsulfonyl)-5-(difluoromethyl)-4,5-dihydro-1*H-*pyrazol | Vinylsulfon | CF₂H |
| 53 | 3-(Ethylsulfonyl)-5-(difluoromethyl)-4,5-dihydro-1*H*-pyrazol | Vinylsulfon | CF₂H |
| 54 | 3-(Benzylsulfonyl)-5-(difluoromethyl)-4,5-dihydro-1*H*-pyrazol | Vinylsulfon | CF₂H |
| 55 | 3-(Phenylsulfonyl)-5-(difluoromethyl)-4,5-dihydro-1*H*-pyrazol | Vinylsulfon | CF₂H |
| 56 | 3-((4-Methylphenyl)sulfonyl)-5-(difluoromethyl)-4,5-dihydro-1*H*-pyrazol | Vinylsulfon | CF₂H |
| 57 | 3-((4-Fluorophenyl)sulfonyl)-5-(difluoromethyl)-4,5-dihydro-1*H*-pyrazol | Vinylsulfon | CF₂H |
| 58 | 3-((4-Chlorophenyl)sulfonyl)-5-(difluoromethyl)-4,5-dihydro-1*H*-pyrazol | Vinylsulfon | CF₂H |
| 59 | 3-((4-Bromophenyl)sulfonyl)-5-(difluoromethyl)-4,5-dihydro-1*H*-pyrazol | Vinylsulfon | CF₂H |
| 60 | 3-((4-Methoxyphenyl)sulfonyl)-5-(difluoromethyl)-4,5-dihydro-1*H*-pyrazol | Vinylsulfon | CF₂H |
| 61 | 3-((3-Methylphenyl)sulfonyl)-5-(difluoromethyl)-4,5-dihydro-1*H*-pyrazol | Vinylsulfon | CF₂H |
| 62 | 3-((2-Methylphenyl)sulfonyl)-5-(difluoromethyl)-4,5-dihydro-1*H*-pyrazol | Vinylsulfon | CF₂H |
| 63 | 3-(Methylsulfonyl)-5-(methyl)-5-(trifluoromethyl)-4,5-dihydro-1*H*-pyrazol | Vinylsulfon | verzweigtes Alkyl |
| 64 | 3-(Phenylsulfonyl)-5-(methyl)-5-(trifluoromethyl)-4,5-dihydro-1*H*-pyrazol | Vinylsulfon | verzweigtes Alkyl |
| 65 | 3-(Methylsulfonyl)-5-phenethyl-5-(trifluoromethyl)-4,5-dihydro-1*H*-pyrazol | Vinylsulfon | verzweigtes Alkyl |
| 66 | 3-(Methylsulfonyl)-5-(phenyl)-5-(trifluoromethyl)-4,5-dihydro-1*H*-pyrazol | Vinylsulfon | verzweigtes Alkyl |
| 67 | 3-(Methylsulfonyl)-5-(4-Methylphenyl)-5-(trifluoromethyl)-4,5-dihydro-1*H-*pyrazol | Vinylsulfon | verzweigtes Alkyl |
| 68 | 3-(Methylsulfonyl)-5-(4-Fluorophenyl)-5-(trifluoromethyl)-4,5-dihydro-1*H-*pyrazol | Vinylsulfon | verzweigtes Alkyl |

Wie man der Tabelle 4 entnimmt, erlaubt das Verfahren eine effiziente Synthese fluoroalkylsubstituierten und sulfon-substituierten Pyrazolen und Pyrazolinen. Wesentlich ist hierfür die kontinuierliche Herstellung der fluorierten Diazoverbindung in guter Ausbeute im ersten Schritt.

### Beispiel 69

### Herstellung von 3-(phenylsulfonyl)-5-(difluoromethyl)-4,5-dihydro-1H-pyrazol

### a) Herstellung des fluorierten Diazoalkans

Es wurden zwei Stammlösungen vorbereitet. 2,2-Difluoroethylamin (0,4 M, 4 Äq) wurde in CHCl₃ gelöst. Tert-Butylnitrit (0,48 M, 4,8 Äq) und 5 mol% Essigsäure (0,16 M, 0,2 Äq) wurden ebenfalls in CHCl₃ gelöst. Beide Lösungen wurden jeweils in einer Spritze vorgelegt und mittels Spritzenpumpe mit einer Flussrate von je 50 µL/min über einen Mikromischer (Little Things Factory, MR Lab Typ MST) mit einem Volumen von 0,2 mL und einen Mikroreaktor (CS Chromatographie PTFE tubing, Art Nr. 590515, PTFE Schlauch, ID = 0,8 mm, Länge 1,6 m) mit einem Volumen von 0,8 mL addiert. Der Mikromischer und der Mikroreaktor wurden auf 55°C erwärmt. Die Mischung der Reaktanden hatte eine Verweilzeit von 10 Minuten im Mikroreaktor.

### b) Umsetzen des fluorierten Diazoalkans mit einem Dipolarophil in einer 1,3-dipolaren Cycloaddition in einem zweiten Mikroreaktor

Eine dritte Stammlösung wurde vorbereitet: Phenyl Vinyl Sulfon (0,1 M, 1 Äq) wurde in CHCl₃ gelöst. Diese Lösungen wurde in einer Spritze vorgelegt und mittels Spritzenpumpe mit einer Flussrate von 50 µL/min zusammen mit dem Schlauchende des ersten Reaktors an einen zweiten Mikromischer angeschlossen (Little Things Factory, MR Lab Typ MST) und einen Mikroreaktor (CS Chromatographie PTFE tubing, Art Nr. 590515, PTFE Schlauch, ID = 0,8 mm, Länge 2,6 m) mit einem Volumen von 1,3 mL. Der Mikromischer und der Mikroreaktor wurden auf 55°C erwärmt. Das Schlauchende des zweiten Reaktors wurde an ein Rückdruckventil (20 psi) angeschlossen und anschließend in einen Rundkolben geleitet. Nach beendeter Zugabe der Reaktanden in die Mikromischer / -reaktoren wurde die aufgesammelte Reaktionslösung im Vakuum vom Lösemittel befreit und der Rückstand säulenchromatographisch aufgereinigt und 3-(phenylsulfonyl)-5-(difluoromethyl)-4,5-dihydro-1*H*-pyrazol erhalten.

### Beispiel 70

### Gramm-weise Herstellung von 3-(Phenylsulfonyl)-5-(difluoromethyl)-4,5-dihydro-1H-pyrazol

### a) Herstellung des fluorierten Diazoalkans

Es wurden zwei Stammlösungen vorbereitet. 2,2-Difluoroethylamin (0,4 M, 4 Äq) wurde in CHCl₃ gelöst. Tert-Butylnitrit (0,48 M, 4,8 Äq) und 5 mol% Essigsäure (0,02 M, 0,2 Äq) wurden ebenfalls in CHCl₃ gelöst. Insgesamt wurden 50 mL von beiden Stammlösungen (entspricht 20 mmol 2,2-Difluorethylamin) vorbereitet. Verglichen mit der Herstellung von 3-(Phenylsulfonyl)-5-(difluoromethyl)-4,5-dihydro-1*H*-pyrazol gemäß Beispiel 40 war die Konzentration der Aminlösung verdoppelt. Beide Lösungen wurden bei Raumtemperatur (20±2°C) jeweils in einer Spritze vorgelegt und mittels Spritzenpumpe mit einer Flussrate von je 50 µL/min über einen Mikromischer (Little Things Factory, MR Lab Typ MST) mit einem Volumen von 0,2 mL und einen Mikroreaktor (CS Chromatographie PTFE tubing, Art Nr. 590515, PTFE Schlauch, ID = 0,8 mm, Länge 1,6 m) mit einem Volumen von 0,8 mL addiert. Der Mikromischer und der Mikroreaktor wurden auf 55°C erwärmt. Die Mischung der Reaktanden hatte eine Verweilzeit von 10 Minuten im Mikroreaktor.

Bei einer Flußrate von 50µL/min, betrug die Additionszeit für die verwendeten Volumina von jeweils 50 mL insgesamt 16,6 Stunden.

### b) Umsetzen des fluorierten Diazoalkans mit einem Dipolarophil in einer 1,3-dipolaren Cycloaddition

Das Schlauchende des Reaktors wurde in einen Kolben geleitet, in dem 1 Äq Phenyl Vinylsulfon (5 mmol), gelöst in Chloroform, vorgelegt wurde. Nach beendeter Zugabe wurde die Reaktionslösung für weitere 24 Stunden bei Raumtemperatur (20±2°C) gerührt. Das Lösemittel wurde im Vakuum entfernt und der Rückstand säulenchromatographisch aufgereinigt. Es wurden 1,08 g 3-(phenylsulfonyl)-5-(difluoromethyl)-4,5-dihydro-1*H-*pyrazol erhalten.

Die erhaltene Menge an Produkt zeigt, dass fluoriertes Diazoalkan in Gramm-Mengen für die Synthese in Schritt b) dargestellt wurde. Für die Gram-Weise Darstellung von 3-(phenylsulfonyl)-5-(difluoromethyl)-4,5-dihydro-1*H*-pyrazol wurde eine Additionszeit von ca. 17 Stunden benötigt. Das kontinuierlich hergestellte fluorierte Diazoalkan akkumulierte jedoch nicht, wodurch die Sicherheit der Synthese auch bei den verwendeten hohen Konzentrationen, Volumina und Reaktionszeiten gewährleistet war.

### Vergleichsbeispiel 71

### Herstellung von 2,2-Difluormethyl-diazomethan unter gemeinsamer Vorlage der Edukte

In einem Vergleichsversuch wurden Difluorethylamin und tert.-Butylnitrit gemeinsam bei Raumtemperatur vorgelegt. In einem 50 mL Kolben wurde Difluorethylamin (1 Äq) in Chloroform vorgelegt. 5 mol% Essigsäure und tert.-Butylnitrit (1,2 Äq) wurden hinzugefügt und die Reaktionsmischung bei Raumtemperatur (20±2°C) gerührt. Proben der Reaktionslösung wurden in regelmäßigen Abständen entnommen und mittels NMR untersucht. Die nachstehende Tabelle 5 enthält Datenpunkte aus diesem Versuch und gibt Aufschluss über den Stoffmengenanteil an Amin bzw. Diazoverbindung in der vorgelegten Lösung.

**Tabelle 5: Stoffmengenanteil an Amin bzw. Diazoverbindung unter gemeinsamer Vorlage der Edukte**

| Zeit | Amin | Diazoverbindung |
|---|---|---|
| 30 min | 93% | 2% |
| 120 min | 72% | 4% |
| 180 min | 26% | 3% |

Wie man der Tabelle 5 entnimmt, war bereits innerhalb von drei Stunden die Stoffmenge des Eduktes Difluorethylamin deutlich gesunken. Die gewünschte Diazoverbindung entstand nur in geringem Umfang. Es wird angenommen, dass eine so genannte background-Reaktion zu einem vorzeitigen Verbrauch des Amins und damit einer Verringerung der Ausbeute an Diazoverbindung führte. Dieser Versuch zeigt, dass das gemeinsame Vorlegen aller drei Edukte Amin, Nitrit und Essigsäure in einem Gefäß bei Raumtemperatur für Zugabezeiten in den Mikroreaktor größer als 2 Stunden nicht geeignet ist.

### Vergleichsbeispiel 72

### Herstellung von 2,2-Difluormethyl-diazomethan unter gemeinsamer Vorlage der Edukte

In weiteren Vergleichsversuchen im Batch-Verfahren wurden jeweils 162 mg (2 mmol) 2,2-Difluorethylamin, 2,4 mmol tert.-Butylnitrit (1,2 Äq) und 0,2 mmol Essigsäure (5 mol%) in 2 ml CDCl₃ gemeinsam vorgelegt. Weiterhin wurde α, α, α-Trifluortoluol als interner Standard zugefügt und die Reaktionsgefäße verschlossen. Ein Vergleichsansatz wurde bei Raumtemperatur (20±2°C) gerührt und Proben wurden nach 30, 60, 120 und 180 Minuten entnommen. Weitere Vergleichsansätze wurden bei 55°C für 5, 15, 30, 60, 120 und 180 Minuten gerührt, und nach der jeweiligen Reaktionszeit kurz im Eisbad gekühlt, bevor eine Probe entnommen und mittels NMR untersucht.

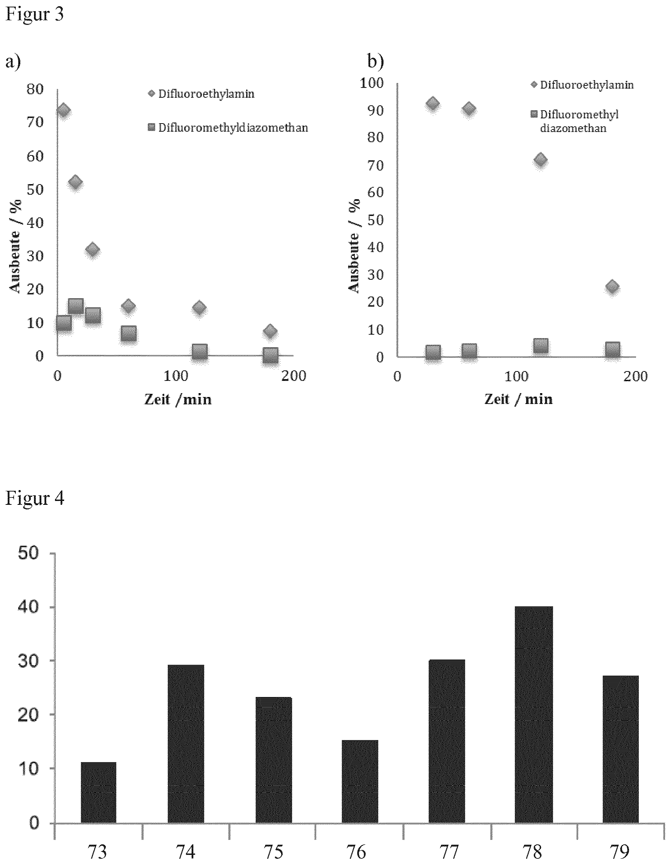

Die Figuren 3a) und 3b) zeigen die jeweiligen Ausbeuten an Difluormethyldiazomethan sowie die Restmengen an Difluorethylamin für die Versuche bei 55°C und bei Raumtemperatur. Wie man der Figur 3a) entnehmen kann, nahm die Menge des Difluorethylamins bei 55°C rasch ab, während lediglich geringe Mengen an Difluormethyldiazomethan hergestellt wurden. Die höchste Ausbeute von 15% wurde nach einer Reaktionszeit von 15 Minuten erzielt. Bei Raumtemperatur wurden lediglich Spuren an Difluormethyldiazomethan nachgewiesen, während die Ausgangsmenge an Difluorethylamin nach 2 Stunden ebenfalls auf ca. 70% absank.

Dies zeigt, dass bei einem gemeinsamen Vorlegen der Edukte Amin, Nitrit und Essigsäure in einem Gefäß bei 55°C die maximale Ausbeute von Difluormethyldiazomethan bei 15% nach 15 Minuten lag. Lagen alle Edukte gemeinsam bei Raumtemperatur vor, so entstanden nur Spuren von Difluormethyldiazomethan. Sowohl bei Raumtemperatur als auch bei 55 °C zersetzen sich die Edukte unter diesen Bedingungen.

Dies illustriert, dass weder bei höheren Temperaturen noch bei Raumtemperatur für eine technische Herstellung ausreichend hohe Ausbeuten erzielt werden können, wenn die Edukte β,β-Difluoralkylamin und Nitrit gemeinsam in einem Gefäß vorgelegt werden. Ein kontinuierliches Verfahren ermöglicht es demgegenüber, über einen längeren Zeitraum beständig hohe Ausbeuten zu erzielen, die beispielsweise weitere chemische Transformationen zu ermöglichen.

### Beispiele 73-79

### Herstellung von Difluormethyl-diazomethan unter kontinuierlichem Durchfluss

In wasserfreiem, entgastem CHCl₃ wurden 0,2 M, 2,2-Difluorethylamin gelöst. In einem weiteren Gefäß wurden 0,24 M tert.-Butylnitrit und 0,08 M Essigsäure in wasserfreiem, entgastem CHCl₃ gelöst. Beide Lösungen wurden jeweils in Spritzen gefüllt, in eine Spritzenpumpe (Chemyx Fusion V710) verbracht und mittels PTFE-Schläuchen mit einem Mikromixer mit einem internen Volumen von 200 µL (Little Things Factory, MR-LAB Type MST) verbunden. Der Mikromixer diente ebenfalls als Mikroreaktor, wobei mittels eines Rückdruckventils (IDEX back pressure assembly) ein Druck von 20 psi erzeugt wurde. Am Auslass wurde das jeweilige Reaktionsgemisch entnommen und in einem mittels NMR untersucht.

Proben unter Verwendung von 10, 20 oder 40 mol-% Essigsäure wurden mit jeweils konstanten Flussraten von 13, 25 oder 50 uL/min in den Reaktor eingebracht, der auf Temperaturen von 55°C, 75°C oder 90°C erwärmt wurde. In der nachstehenden Tabelle 6 sind die jeweiligen Beispiele zusammengefasst.

**Tabelle 6: Beispiele 73-79**

| Beispiel | Flussrate [µL/min] | Temperatur [°C] | Essigsäure [mol-%] |
|---|---|---|---|
| 73 | 50 | 55 | 10 |
| 74 | 50 | 75 | 10 |
| 75 | 25 | 75 | 10 |
| 76 | 13 | 75 | 10 |
| 77 | 50 | 75 | 20 |
| 78 | 50 | 75 | 40 |
| 79 | 50 | 90 | 40 |

Die Figur 4 zeigt die jeweilig erzielten Ausbeuten an Difluormethyldiazomethan angegeben in % auf der Y-Achse. Wie man der Figur 4 entnehmen kann, konnte im Unterschied zur Batch Reaktion eine Ausbeute von mehr als 10% in allen Versuchen kontinuierlich erzielt werden. Das Reaktionsgemisch kann weiterhin direkt zu Folgereaktionen hinzuaddiert werden. Die Skalierbarkeit kann dadurch erreicht werden, dass der Volumenstrom über einen längeren Zeitraum addiert wird, oder durch Parallelisierung der Mikroreaktoren.

## Patentansprüche

1. Verfahren zur Herstellung eines fluorierten Diazoalkans, wobei es sich bei dem Verfahren um ein kontinuierliches Verfahren handelt und ein β,β-Difluoralkylamin mit einem organischen Nitrit in einem Reaktor umgesetzt wird, **dadurch gekennzeichnet, dass** das β,β-Difluoralkylamin und das organische Nitrit in getrennten Gefäßen vorgelegt werden.

2. Verfahren nach Anspruch 1, **dadurch gekennzeichnet, dass** man das β,β-Difluoralkylamin in einem ersten Gefäß vorlegt und das organische Nitrit und Essigsäure gemeinsam in einem zweiten Gefäß vorlegt, oder das organische Nitrit in einem ersten Gefäß vorlegt und das β,β-Difluoralkylamin und Essigsäure gemeinsam in einem zweiten Gefäß vorlegt.

3. Verfahren nach Anspruch 1 oder 2, **dadurch gekennzeichnet, dass** man die Reaktion des β,β-Difluoralkylamins mit dem organischen Nitrit im Reaktor bei einer Temperatur im Bereich von ≥ 40°C bis ≤ 100°C, bevorzugt im Bereich von ≥ 40°C bis ≤ 80°C, vorzugsweise bei einer Temperatur im Bereich von ≥ 55°C bis ≤ 75°C, durchführt.

4. Verfahren nach einem der vorherigen Ansprüche, **dadurch gekennzeichnet, dass** die Verweilzeit der Komponenten β,β-Difluoralkylamin, organisches Nitrit und Essigsäure im Reaktor in einem Bereich von ≥ 30 Sekunden bis ≤ 1 Stunde, vorzugsweise im Bereich von ≥ 1 min bis ≤ 20 min, bevorzugt im Bereich von ≥ 2 min bis ≤ 10 min, liegt.

5. Verfahren nach einem der vorherigen Ansprüche, **dadurch gekennzeichnet, dass** man das organische Nitrit in einem ≥ 0,5-fachen bis ≤ 2-fachen, vorzugsweise einem ≥ 1-fachen bis ≤ 1,5-fachen, bevorzugt einem ≥ 1-fachen bis ≤ 1,2-fachen, molaren Überschuss bezogen auf das β,β-Difluoralkylamin, verwendet.

6. Verfahren nach einem der vorherigen Ansprüche, **dadurch gekennzeichnet, dass** der Stoffmengenanteil der Essigsäure im Bereich von ≥ 1 mol% bis ≤ 50 mol%, vorzugsweise im Bereich von ≥ 5 mol% bis ≤ 40 mol%, bevorzugt im Bereich von von ≥ 5 mol% bis ≤ 10 mol%, bezogen auf die Stoffmenge des β,β-Difluoralkylamins, liegt.

7. Verfahren nach einem der vorherigen Ansprüche, **dadurch gekennzeichnet, dass** das β,β-Difluoralkylamin die folgende allgemeinen Formel (1) aufweist: 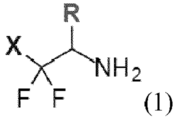 worin:
R ist ausgewählt aus der Gruppe umfassend H und/oder substituiertes oder unsubstituiertes Alkyl, Aryl, Arylalkyl, Cyclyl, Heteroaryl oder Heterocyclyl,
X ist ausgewählt aus der Gruppe umfassend H, F, Cl, CN, CO₂R', CONR', COR', SO₂R', SO₂NR'₂ und/oder substituiertes oder unsubstituiertes Alkyl, Aryl, Arylalkyl, Cyclyl, Heteroaryl oder Heterocyclyl, und
R' ist gegebenenfalls unabhängig voneinander ausgewählt aus der Gruppe umfassend H und/oder substituiertes oder unsubstituiertes Alkyl, Aryl, Arylalkyl, Cyclyl, Heteroaryl oder Heterocyclyl.

8. Verwendung des Verfahren nach einem der Ansprüche 1 bis 7 zur Herstellung einer fluoralkyl-substituierten Verbindung, insbesondere eines di- oder trifluoralkyl-substituierten Pyrazols, Pyrazolins oder Cyclopropans.

9. Verfahren zur Herstellung einer fluoralkyl-substituierten Verbindung, insbesondere eines di- oder trifluoralkyl-substituierten Pyrazols, Pyrazolins oder Cyclopropans, wobei das Verfahren die folgenden Schritte umfasst:
a) Herstellen eines fluorierten Diazoalkans gemäß dem Verfahren nach einem der Ansprüche 1 bis 7, und
b) Umsetzen des fluorierten Diazoalkans in einer Cyclopropanierung oder mit einem Dipolarophil in einer 1,3-dipolaren Cycloaddition.

10. Verfahren nach Anspruch 9, **dadurch gekennzeichnet, dass** das Dipolarophil folgende allgemeinen Formel (2) oder (3) aufweist: 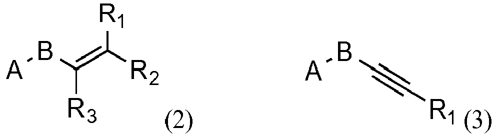 worin:
A ist ausgewählt aus der Gruppe umfassend substituiertes oder unsubstituiertes Alkyl, Aryl, Arylalkyl, Cyclyl, Heteroaryl, Heterocyclyl, Alkoxy, Aryloxy, NR"₂ und/oder NHR",
B ist ausgewählt aus der Gruppe umfassend CO und/oder SO₂,
R₁, R₂, R₃ sind jeweils unabhängig voneinander ausgewählt aus der Gruppe umfassend H, F, Cl, C₁₋₃-Alkyl, CF₃, CF₂H, CFH₂, Phenyl, COR'" und/oder SO₂R"',
R" ist gegebenenfalls unabhängig voneinander ausgewählt aus der Gruppe umfassend C₁₋₈-Alkyl, Phenyl und/oder C₅₋₆-Heteroaryl, und
R'" ist ausgewählt aus der Gruppe umfassend H und/oder substituiertes oder unsubstituiertes Alkyl, Aryl, Arylalkyl, Cyclyl, Heteroaryl oder Heterocyclyl,
wobei im Fall des Dipolarophils der Formel (2) A und R₁ gemeinsam einen 5-, 6- oder 7-gliedrigen Ring ausbilden können, der als Keton, Lacton, Lactam, Sulfon, Sulfonsäurester, Sultam, Anhydrid oder Imid vorliegt.
